# EUROPÄISCHE PATENTSCHRIFT

(11) **EP 2 157 995 B1**
(45) Veröffentlichungstag und Bekanntmachung des Hinweises auf die Patenterteilung: **16.03.2016**
(21) Anmeldenummer: 08773459.6
(22) Anmeldetag: 16.06.2008
(51) Int. Cl.: A61M 39/12

(54) **SPANNADAPTER FÜR EINEN KATHETER**
CLAMP ADAPTER FOR A CATHETER
ADAPTATEUR DE SERRAGE POUR CATHÉTER

(30) Priorität: 22.06.2007 DE 102007029229
(43) Veröffentlichungstag der Anmeldung: 03.03.2010
(73) Patentinhaber: Pajunk GmbH & Co. KG Besitzverwaltung, 78187 Geisingen (DE)
(72) Erfinder: PAJUNK, Horst, 78187 Geisingen (DE); PAJUNK, Heinrich, 78187 Geisingen (DE)
(74) Vertreter: Westphal, Mussgnug & Partner Patentanwälte mbB
(86) Internationale Anmeldenummer: PCT/EP2008/004825
(87) Internationale Veröffentlichungsnummer: WO 2009/000439

(56) Entgegenhaltungen:
- WO-A-91/12840
- DE-C1- 10 100 975
- DE-U1- 20 018 390
- DE-U1- 20 305 061
- US-A- 4 187 848
- US-A1- 2004 039 373
- US-B1- 6 572 590

## Beschreibung

Die Erfindung betrifft einen Spannadapter für einen Katheter gemäß dem Oberbegriff des Patentanspruchs 1.

In der Anästhesie werden für länger dauernde operative Eingriffe, für die postoperative Schmerztherapie und für die Behandlung chronischer Schmerzzustände die einen gewissen Körperbereich versorgenden Nerven durch Einleiten eines Anästhetikums blockiert. Zum Einleiten des Anästhetikums oder auch einer sonstigen Flüssigkeit wird ein Katheter benutzt, dessen distales Ende möglichst nahe an dem Nerv positioniert wird, um mit einer möglichst geringen Menge des Anästhetikums eine optimale Wirkung zu erzielen. Damit der Katheter in der gewünschten Lage platziert werden und im Bedarfsfalle auch über eine längere Zeit verbleiben kann, besteht der Katheter aus einem langen dünnen flexiblen Kunststoffschlauch.

Um den flexiblen Katheter in die Nervenscheide- oder den Nervenkanal einführen zu können, wird eine Kanüle verwendet, die in die Nervenscheide bzw. den Nervenkanal eingesetzt wird und durch die dann der Katheter eingeführt wird. Um über den Katheter eine Flüssigkeit z.B. ein Anästhetikum zu applizieren, wird an das extrakorporal verbleibende proximale Ende des Katheters eine Spritze angesetzt. Hierzu dient ein an dem proximalen Ende des Katheters angebrachter Adapter. Soll der Katheter über einen längeren Zeitraum in seiner Lage verbleiben, so muss die zum Einführen des Katheters dienende Kanüle abgezogen werden. Hierzu ist es notwendig, den Adapter von dem Katheter zu entfernen, damit die Kanüle über das proximale Ende des Katheters abgezogen werden kann. Um über den liegenden Katheter das Anästhetikum nachdosieren zu können, muss der Adapter nach dem Abziehen der Kanüle wieder an dem Ende des Katheters angebracht werden.

Um den Adapter in einfacher Weise am Ende des Katheters lösbar anzubringen, ist aus der DE 101 00 975 C1 ein Spannadapter bekannt, bei dem in einen Spannkörper eine hohlzylindrische formelastische Spannbuchse eingesetzt ist. Das proximale Ende des Katheters wird koaxial in diese Spannbuchse eingeführt. Daraufhin wird die Spannbuchse durch einen auf den Spannkörper aufgesetzten Spanndeckel axial gestaucht, wodurch sich der Innendurchmesser der Spannbuchse verkleinert und der Katheter festgelegt und an seinem Außenumfang abgedichtet wird. Bei diesem bekannten Spannadapter ist zum Stauchen der Spannbuchse der Spanndeckel mittels eines Schraubgewindes axial auf dem Spannkörper bewegbar.

Aus der US 6 572 590 B1 ist ein Spannadapter für einen Katheter gemäß dem Oberbegriff des Patentanspruchs 1 bekannt. In einen Spannkörper ist eine formelastische Spannbuchse einsetzbar, in deren Innenkanal der Katheter eingeführt wird. Die Spannbuchse kann in dem Spannkörper mittels eines Stößels axial gestaucht werden, um den Katheter zu spannen und in dem Spannadapter abgedichtet festzulegen. Der Stößel ist an einem Spanndeckel angebracht, der in ein Gehäuse eingesetzt ist, welches axial auf dem Spannkörper verschiebbar ist. Eine den Spannkörper umschließende Schraubenfeder drückt das Gehäuse mit dem Spanndeckel und dem Stößel gegen die Spannbuchse, um diese zu stauchen. An dem Spannkörper ist ein Spannhebel schwenkbar gelagert, der in einer Spannstellung von dem Spannkörper abgeschwenkt ist und das Gehäuse freigibt, so dass dieses durch die Schraubenfeder in die Spannstellung gedrückt werden kann. Wird der Spannhebel in eine an dem Spannkörper anliegende Offenstellung geschwenkt, so drückt der Spannhebel das Gehäuse gegen die Kraft der Schraubenfeder in eine die Spannbuchse axial entlastende Stellung des Stößels.

Aus der US 2004/0039373 A1 ist ein Spannadapter für einen Katheter bekannt, bei welchem in einen Spannkörper eine formelastische Spannbuchse eingesetzt ist, in deren Innenkanal der Katheter eingeführt wird. An dem Spannkörper ist ein Spannhebel schwenkbar gelagert, der in einer an den Spannkörper angeschwenkten Spannstellung gegen einen axial in dem Spannkörper geführten Stößel drückt, welcher wiederum die Spannbuchse axial staucht, um den Katheter zu spannen. Wird der Spannhebel von dem Spannkörper in eine Offenstellung abgeschwenkt, so gibt der Spannhebel den Stößel frei, so dass die Spannbuchse aufgrund ihrer Eigenelastizität ihre ursprüngliche, den Katheter freigebende Form annehmen kann.

Aus der EP 1 033 146 B1 ist ein Spannadapter für einen Katheter bekannt, der zwei gegeneinander verschwenkbare und miteinander verrastbare Backen aufweist, zwischen denen ein Schlauchstück angeordnet ist, welches das proximale Ende des Katheters aufnimmt.

Der Erfindung liegt die Aufgabe zugrunde, einen Spannadapter zur Verfügung zu stellen, der ein einfaches und zuverlässiges Fixieren des Katheterendes in dem Spannadapter ermöglicht.

Diese Aufgabe wird erfindungsgemäß gelöst durch einen Spannadapter mit den Merkmalen des Patentanspruchs 1.

Vorteilhafte Ausführungen und Weiterbildungen der Erfindung sind in den Unteransprüchen angegeben.

Bei dem erfindungsgemäßen Spannadapter wird das proximale Ende des Katheters in einer weichelastischen Spannbuchse aufgenommen und durch axiales Stauchen der Spannbuchse fixiert. Zum Stauchen der Spannbuchse dient ein Spannhebel, der um eine zur Längsachse des Spannadapters quer verlaufende Schwenkachse schwenkbar ist. Bei dieser Schwenkbewegung bewirkt der Spannhebel mittels einer Kulissenführung eine gegenseitige axiale lineare Verschiebung des Spanndeckels und des Spannkörpers. Durch ein einfaches Verschwenken des Spannhebels kann somit der Spannadapter von einer Offenstellung in eine Spannstellung und umgekehrt gebracht werden. In der Offenstellung ist die Spannbuchse axial entlastet, so dass der Katheter eingeführt werden kann bzw. der Spannadapter vom Katheterende abgezogen werden kann. In der Spannstellung ist die Spannbuchse axial gestaucht und klemmt das proximale Ende des Katheters an dessen Außenumfang. Die beiden Schwenkstellungen des Spannhebels sind eindeutig mechanisch definiert, so dass insbesondere das Befestigen des Spannadapters auf dem Katheterende in einfacher und zuverlässiger Weise allein durch Verschwenken des Spannhebels in seine Endlage in der Spannstellung möglich ist.

Der Spannhebel ist vorzugsweise schwenkbar an dem Spanndeckel gelagert, wobei die Kulissenführung zwischen dem Schwenkhebel und dem Spannkörper ausgebildet ist. Selbstverständlich ist auch eine schwenkbare Lagerung des Spannhebels an dem Spannkörper möglich, wobei dann die Kulissenführung zwischen dem Spannhebel und dem Spanndeckel ausgebildet ist.

In einer vorteilhaften Ausführung ist die Kulissenführung durch eine schräg zur Längsachse verlaufende Führungsbahn und einen in dieser Führungsbahn laufenden Führungszapfen gebildet. Vorzugsweise ist die Führungsbahn in dem Spannhebel, z.B. als Längsschlitz und der Führungszapfen entsprechend an dem Spannkörper bzw. dem Spanndeckel ausgebildet. Selbstverständlich ist auch hier eine umgekehrte Ausbildung der Führungsbahn an dem Spannkörper bzw. dem Spanndeckel und des Führungszapfens an dem Spannhebel möglich.

Zweckmäßig ist es, wenn der Spannhebel mit der Kulissenführung so ausgebildet ist, dass er in der Offenstellung von dem Spannadapter abgespreizt ist und in der Spannstellung an dem Spannadapter anliegt. Damit ist gewährleistet, dass der Spannhebel bei fixiertem Katheter nicht stört und nicht unbeabsichtigt in die Offenstellung verschwenkt wird.

Der Spannadapter kann für einen einfachen Katheter und ebenso für einen Stimulationskatheter ausgebildet sein. In der Ausführung für einen Stimulationskatheter weist der Spannkörper proximal an die Spannbuchse anschließend eine Kontaktbuchse auf, die bei eingesetztem Katheter einen elektrisch leitenden Stimulationsdraht des Katheters kontaktiert und über einen Steckverbinder mit einer elektrischen Stimulationseinrichtung verbunden werden kann.

Im Folgenden wird die Erfindung anhand von in der Zeichnung dargestellten Ausführungsbeispielen näher erläutert. Es zeigen
- Figur 1: eine Seitenansicht des Spannadapters in einer ersten Ausführung in der Spannstellung,
- Figur 2: eine Stirnansicht auf das distale Ende des Spannadapters,
- Figur 3: eine Seitenansicht des Spannadapters in der Offenstellung,
- Figur 4: einen Axialschnitt des Spannadapters in der Offenstellung,
- Figur 5: einen Axialschnitt des Spannadapters in der Spannstellung,
- Figur 6: eine perspektivische Explosionsdarstellung des Spannadapters,
- Figur 7: eine Seitenansicht des Spannadapters in Explosionsdarstellung,
- Figur 8: eine Seitenansicht des Spannadapters in einer zweiten Ausführung in der Spannstellung,
- Figur 9: eine Seitenansicht des Spannadapters in der zweiten Ausführung in der Offenstellung,
- Figur 10: einen Axialschnitt des Spannadapters in der zweiten Ausführung in der Offenstellung und
- Figur 11: einen Axialschnitt des Spannadapters in der zweiten Ausführung in der Spannstellung.

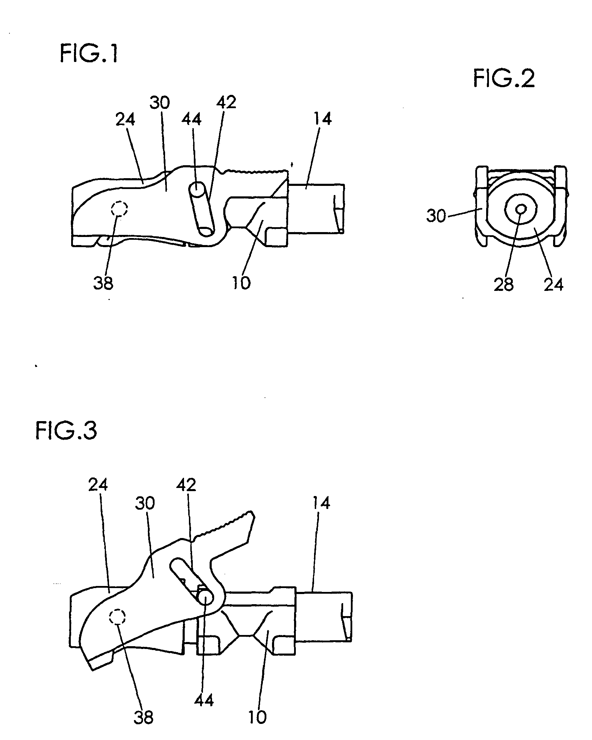

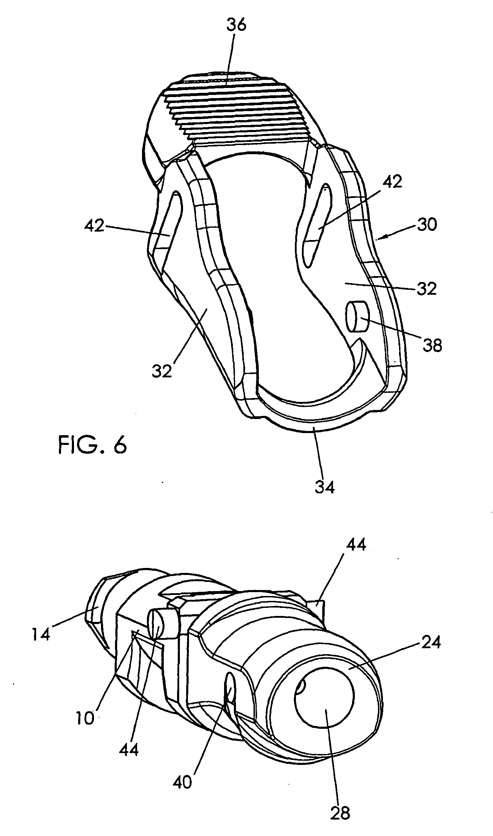

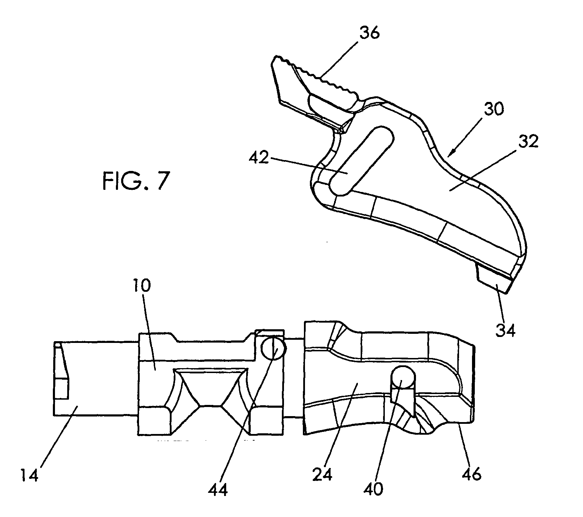

In den Figuren 1 bis 7 ist der Spannadapter in einer ersten Standardausführung für einen einfachen Katheter gezeigt.

Der Spannadapter weist einen Spannkörper 10 auf, der vorzugsweise ein Kunststoff-Formteil ist. Der Spannkörper 10 weist eine axial durchgehende Bohrung 12 auf. Das proximale Ende des Spannkörpers 10 ist als Spritzenansatz 14 ausgebildet, z.B. als Luer-Lock-Ansatz. Die Bohrung 12 mündet mit ihrem proximalen Ende koaxial in den Spritzenansatz 14. Distal erweitert sich die Bohrung 12 zu einem koaxial fluchtenden Aufnahmeraum 16. Der Übergang von dem Durchmesser der Bohrung 12 zu dem größeren Durchmesser des Aufnahmeraums 16 bildet eine radiale Innenschulter 18. Der Aufnahmeraum 16 erweitert sich in distaler Richtung von der Innenschulter 18 zunächst leicht konisch und geht dann in einen zylindrischen Abschnitt über.

In den Aufnahmeraum 16 ist eine Spannbuchse 20 eingesetzt, die aus einem formelastischen elastomeren Kunststoff besteht. Die Spannbuchse 20 entspricht in ihrer äußeren Umfangsform der Innenumfangsform des Aufnahmeraums 16 und wird axial mittig von einem Innenkanal 22 durchsetzt, der bei eingesetzter Spannbuchse 20 koaxial fluchtend an die Bohrung 12 des Spannkörpers 10 anschließt. Die in den Aufnahmeraum 16 eingesetzte Spannbuchse 20 sitzt mit ihrer proximalen Stirnfläche an der Innenschulter 18 auf, während die distale Stirnfläche der Spannbuchse 20 gegenüber dem distalen Ende des Aufnahmeraumes 16 nach innen versetzt ist.

Ein Spanndeckel 24, der ebenfalls ein Kunststoff-Formteil ist, ist distal auf den Spannkörper 10 aufgesetzt. Der Spanndeckel 24 übergreift topfförmig das distale Ende des Spannkörpers 10 und ist auf diesem axial verschiebbar geführt. An dem Spanndeckel 24 ist eine Druckfläche 26 ausgebildet, die in Form eines koaxialen zylindrischen Vorsprungs in das distale Ende des Aufnahmeraumes 16 eingreift. Mit der Druckfläche 26 liegt der Spanndeckel 24 an der distalen Stirnfläche der Spannbuchse 20 an. Der Spanndeckel 24 wird mittig axial von einer Einführöffnung 28 durchsetzt, die sich in proximaler Richtung trichterförmig auf den Durchmesser des Innenkanals 22 der Spannbuchse 20 verengt.

An dem Spanndeckel 24 ist ein Spannhebel 30 um eine zur Mittelachse des Spannkörpers 10 senkrecht verlaufende Schwenkachse schwenkbar gelagert. Der Spannhebel 30 ist ebenfalls ein Kunststoff-Formteil. Der Spannhebel 30 weist zwei rechts und links an dem Spanndeckel 24 anliegende Wangen 32 auf, die distal an der Unterseite durch einen Querbügel 34 und proximal an der Oberseite durch einen als Grifffläche ausgebildeten Querbügel 36 miteinander verbunden sind. An den Innenflächen der Wangen 32 sind jeweils Lagerzapfen 38 angeformt, die in Lagerbohrungen 40 in den Seitenflächen des Spanndeckels 24 eingreifen. Die Lagerzapfen 38 und die Lagerbohrungen 40 bilden somit die Schwenkachse, um welche der Spannhebel 30 in Bezug auf den Spanndeckel 24 verschwenkbar ist. Der Spannhebel 30 wird auf den Spanndeckel 24 aufgeschnappt, wozu die Wangen 32 leicht auseinander gespreizt werden, bis die Lagerzapfen 38 in die Lagerbohrungen 40 einschnappen, so dass der Spannhebel 30 unverlierbar und schwenkbar auf dem Spanndeckel 24 gelagert ist.

In dem proximalen Bereich des Spannhebels 30 liegen die Wangen 32 jeweils außen an dem Spannkörper 10 an und sind mit Führungsbahnen 42 ausgebildet. Die Führungsbahnen 42 werden durch Längsschlitze in den Wangen 32 gebildet, die unter einem spitzen Winkel schräg in Bezug auf die Mittelachse des Spannadapters verlaufen, wie am besten aus den Figuren 1 und 3 zu sehen ist. In die Führungsbahnen 42 greifen jeweils Führungszapfen 44 ein, die an den Außenseiten des Spannkörpers 10 angeformt sind. Die Führungsbahnen 42 und die Führungszapfen 44 wirken als Kulissenführung in folgender Weise zusammen.

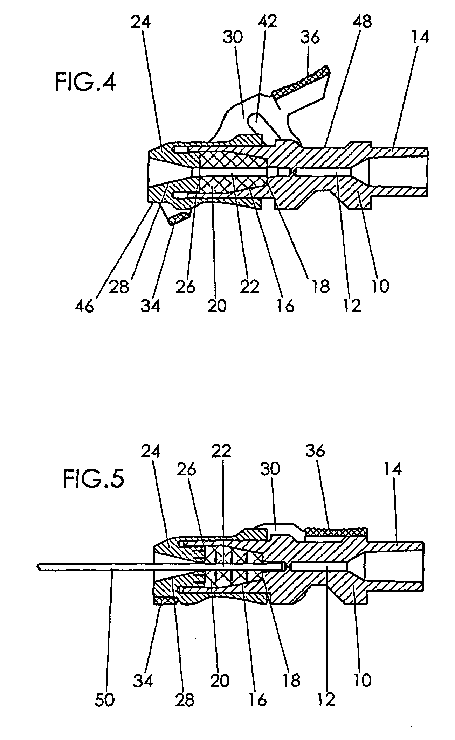

Der Spannhebel 30 kann um seine durch die Lagerzapfen 38 und die Lagerbohrungen 40 gebildete Schwenkachse in eine in den Figuren 3 und 4 gezeigte Offenstellung geschwenkt werden. In dieser Offenstellung ist das proximale Ende des Spannhebels 30 von dem Spannkörper 10 abgespreizt. Die Führungszapfen 44 gelangen in dieser Offenstellung an das untere Ende der Führungsbahnen 42, wobei der Anschlag des unteren Endes der Führungsbahnen 42 an die Führungszapfen 44 die Schwenkbewegung des Spannhebels 30 in der Offenstellung begrenzt. Die Schrägstellung der Führungsbahnen 42 bewirkt dabei, dass der Spanndeckel 24 gegenüber dem Spannkörper 10 axial in distaler Richtung verschoben wird. Die Druckfläche 26 des Spanndeckels 24 bewegt sich dabei in distaler Richtung soweit, dass die Spannbuchse 20 axial entlastet ist und der Spanndeckel 24 auf die Spannbuchse 20 keinen axialen Druck ausübt. Wird der Spannhebel 30 aus der abgespreizten Offenstellung gegen den Spannkörper 10 verschwenkt, so gelangt der Spannhebel 30 in die in den Figuren 1 und 5 gezeigte Spannstellung. In dieser Spannstellung liegt der Spannhebel 30 an dem Spannkörper 10 an, wobei Umfangsaussparungen 46 und 48 des Spannkörpers 10 die Querbügel 34 und 36 aufnehmen. Bei dieser Schwenkbewegung des Spannhebels 30 in die Spannstellung bewegen sich die Führungsbahnen 42 auf den Führungszapfen 44, bis sie mit ihrem oberen Ende an den Führungszapfen 44 anschlagen, wie dies in Figur 1 zu sehen ist. Aufgrund der Schrägstellung der Führungsbahnen 42 wird bei dieser Schwenkbewegung der Spanndeckel 24 axial auf dem Spannkörper 10 in proximaler Richtung gezogen, wie aus den Figuren 1 und 5 zu sehen ist. Dabei drückt der Spanndeckel 24 mit seiner inneren Druckfläche 26 axial gegen die Spannbuchse 20, wie in Figur 5 durch Pfeile angezeigt ist. Da die Spannbuchse 20 proximal durch die Innenschulter 18 abgestützt ist, wird die Spannbuchse 20 durch die Druckfläche 26 axial gestaucht. Da die Spannbuchse 20 an ihrem Außenumfang durch den Aufnahmeraum 16 abgestützt ist, bewirkt diese axiale Stauchung eine Verengung des Innenkanals 22 der elastomeren Spannbuchse 20, wie dies in Figur 5 durch die radial gerichteten Pfeile eingezeichnet ist.

Wird der Spannhebel 30 in seine in den Figuren 3 und 4 gezeigte Offenstellung geschwenkt, so ist die Spannbuchse 20 entlastet und ihr Innenkanal 22 weist einen der Einführöffnung 28 des Spanndeckels 24 entsprechenden Durchmesser auf. Es kann nun ein Katheter 50 durch die Einführöffnung 28 des Spanndeckels 24 in den Innenkanal 22 der Spannbuchse 20 eingeführt werden. Sobald der Katheter 50 durch die Einführöffnung 28 und den Innenkanal 22 eingeführt ist, bis sein proximales Ende in dem Spannkörper 10 mit dessen Bohrung 12 axial fluchtend anschlägt, wird der Spannhebel 30 in seine Spannstellung geschwenkt. Dadurch wird die Spannbuchse 20 axial gestaucht und über ihre gesamte axiale Länge radial von außen gegen den Außenumfang des Katheters 50 gepresst, wie dies in Figur 5 gezeigt ist. Das proximale Ende des Katheters 50 ist dadurch in dem Spannadapter fixiert und durch die Spannbuchse 20 am Außenumfang in dem Spannadapter abgedichtet. Es kann nun über eine an den Spritzenansatz 14 angeschlossene Spritze eine Flüssigkeit, z.B. ein Anästhetikum zugeführt werden. Um den Spannadapter von dem Katheter 50 abzunehmen, muss nur der Spannhebel 30 in die Offenstellung der Figuren 3 und 4 verschwenkt werden. Dadurch wird die Spannbuchse 20 axial entlastet und nimmt aufgrund ihrer Formelastizität wieder ihre ursprüngliche Gestalt an, in welcher der Innenkanal 22 den größeren Durchmesser aufweist, der ein Abziehen von dem Katheter 50 ermöglicht.

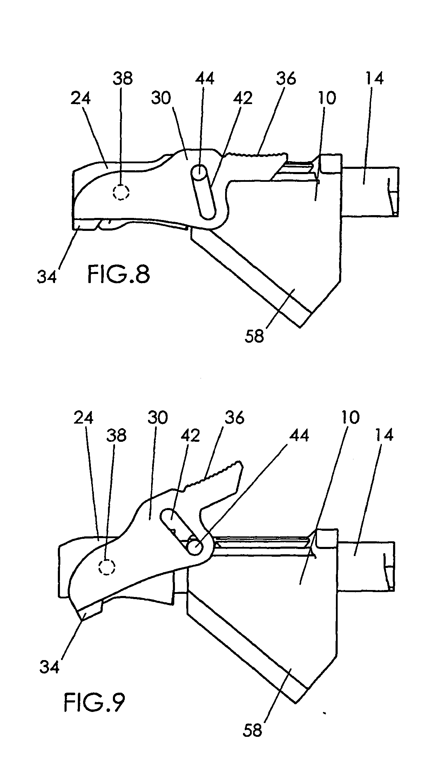

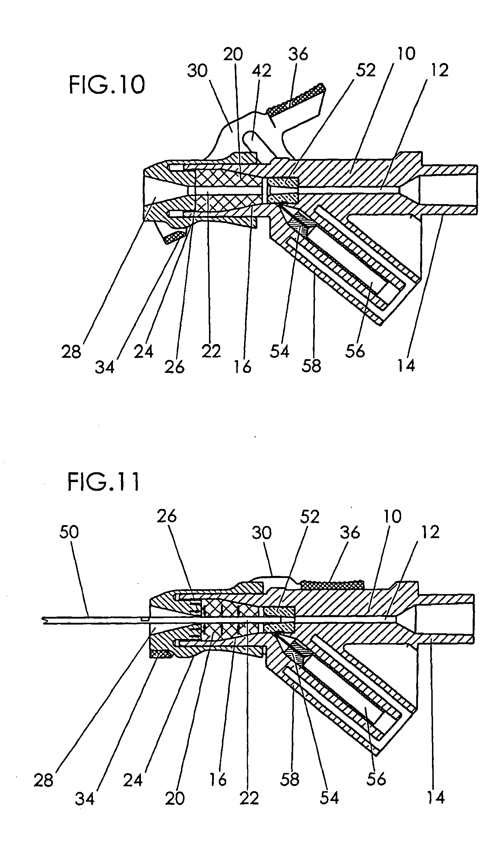

In den Figuren 8 bis 11 ist eine zweite Ausführung des Spannadapters dargestellt, die sich für einen Stimulationskatheter eignet. Der Spannmechanismus entspricht in dieser Ausführung vollständig dem Spannmechanismus der ersten Ausführung, so dass auf die vorangehende Beschreibung Bezug genommen wird.

Zum Unterschied gegenüber der ersten Ausführung schließt sich bei der zweiten Ausführung proximal an den Aufnahmeraum 16 für die Spannbuchse 20 noch eine koaxiale Kontaktbuchse 52 an. Die Kontaktbuchse 52 besteht aus einem elektrisch leitenden Metall und weist eine durchgehende Bohrung auf, die axial mit der Bohrung 12 des Spannkörpers 10 und dem Innenkanal 22 der Spannbuchse 20 fluchtet. Die Kontaktbuchse 52 ist über einen Leiter 54 elektrisch verbunden mit einer Steckverbinderbuchse 56, die in einen seitlichen Ansatz 58 des Spannkörpers 10 eingegossen ist.

Bei einem Stimulationskatheter führt ein elektrisch leitender Draht in dem Katheter 50 bis zu dessen distaler Spitze, um über elektrische Stimulationsimpulse die Position der distalen Katheterspitze lokalisieren zu können. Wird der Stimulationskatheter in den Spannadapter eingesetzt, so wird der elektrisch leitende Draht aus dem proximalen Ende des Katheters 50 herausgeführt und an den Außenumfang des Katheters 50 umgebogen. Nun wird das proximale Ende des Katheters 50 durch die Einführöffnung 28, den Innenkanal 22 der Spannbuchse 20 bis in die Kontaktbuchse 52 eingeschoben, wobei der am Außenumfang des Katheters 50 umgebogene leitende Draht kontaktierend gegen den Innenumfang der Bohrung der Kontaktbuchse 52 gedrückt wird. In dieser Stellung wird der Spannhebel 30 in die Spannstellung verschwenkt, so dass der Katheter 50 in dem Spannadapter fixiert ist und der leitende Draht des Katheters 50 unter Druck an der Kontaktbuchse 52 anliegend gehalten wird. Ein Stimulationsgerät kann nun an die Steckverbinderbuchse 56 angeschlossen werden, um Stimulationsimpulse über die Steckverbinderbuchse 56, den Leiter 54, die Kontaktbuchse 52 und den Draht des Katheters 50 zur distalen Katheterspitze zu führen.

### Bezugszeichenliste

- 10: Spannkörper
- 12: Bohrung
- 14: Spritzenansatz
- 16: Aufnahmeraum
- 18: Innenschulter
- 20: Spannbuchse
- 22: Innenkanal
- 24: Spanndeckel
- 26: Druckfläche
- 28: Einführöffnung
- 30: Spannhebel
- 32: Wangen
- 34: distaler Querbügel
- 36: proximaler Querbügel
- 40: Lagerbohrungen
- 42: Führungsbahnen
- 44: Führungszapfen
- 46: Umfangsaussparung
- 48: Umfangsaussparung
- 50: Katheter
- 52: Kontaktbuchse
- 54: Leiter
- 56: Steckverbinderbuchse
- 58: Ansatz

## Patentansprüche

1. Spannadapter für einen Katheter, mit einem Spannkörper (10), mit einer den Spannkörper (10) axial durchsetzenden Bohrung (12), die sich in einem Spritzenansatz (14) fortsetzt, mit einer in den Spannkörper (10) einsetzbaren formelastischen Spannbuchse (20), deren Innenkanal (22) mit der Bohrung (12) des Spannkörpers (10) kommuniziert und deren Ende in dem Spannkörper (10) axial abgestützt ist, mit einem Spanndeckel (24), der auf den Spannkörper (10) aufsetzbar ist, eine mit dem Innenkanal (22) der Spannbuchse (20) fluchtende Einführöffnung (28) aufweist, axial in Bezug auf den Spannkörper (10) bewegbar ist und an der Stirnfläche der Spannbuchse (20) angreift, wobei der Katheter (50) durch die Einführöffnung (28) des Spanndeckels (24) hindurch in den Innenkanal (22) der Spannbuchse einführbar und in dem Innenkanal (22) durch axiales Stauchen der Spannbuchse (20) mittels des Spanndeckels (24) spannbar ist, wobei der Spanndeckel (24) gegenüber dem Spannkörper (10) linear verschiebbar geführt ist, und mit einem Spannhebel (30), der um eine Schwenkachse, die zur Längsachse des Spannadapters quer verläuft, zwischen einer Offenstellung und einer Spannstellung schwenkbar ist, wobei der Spannhebel (30) mit dem Spanndeckel so zusammenwirkt, dass der Spanndeckel (24) in der Offenstellung die Spannbuchse (20) axial entlastet und in der Spannstellung axial staucht,
**dadurch gekennzeichnet, dass** sich der Spannkörper (10) proximal in dem Spritzenansatz (14) fortsetzt, dass die Spannbuchse (20) distal in den Spannkörper (10) einsetzbar ist, dass der Innenkanal (22) der Spannbuchse (20) mit dem distalen Ende der Bohrung (12) des Spannkörpers (10) kommuniziert, dass das proximale Ende der Spannbuchse (20) in dem Spannkörper (10) axial abgestützt ist, dass der Spanndeckel (24) das distale Ende des Spannkörpers (10) topfförmig übergreift und mit einer koaxialen Druckfläche (26) in das distale Ende des Spannkörpers (10) eingreift und an der distalen Stirnfläche der Spannbuchse (20) anliegt, dass der Spannhebel (30) an dem Spanndeckel (24) schwenkbar gelagert ist und mittels einer zwischen dem Spannhebel (30) und dem Spannkörper (10) wirksamen Kulissenführung (42, 44) den Spanndeckel (24) in Bezug auf den Spannkörper (10) axial bewegt, dass der Spannhebel (30) in seiner Offenstellung von dem Spannadapter abgespreizt ist, wobei der Spanndeckel (24) gegenüber dem Spannkörper (10) axial in distaler Richtung verschoben ist, und dass der Spannhebel (30) in seiner Spannstellung an dem Spannadapter anliegt, wobei der Spanndeckel (24) auf dem Spannkörper (10) axial in proximaler Richtung gezogen wird.

2. Spannadapter nach Anspruch 1, wobei
die Kulissenführung wenigstens eine schräg zur Längsachse des Spannadapters verlaufende Führungsbahn (42) aufweist, in welche ein Führungszapfen (44) eingreift.

3. Spannadapter nach Anspruch 2, wobei
die wenigstens eine Führungsbahn (42) an dem Spannhebel (30) und der wenigstens eine Führungszapfen (44) an dem Spannkörper (10) ausgebildet sind.

4. Spannadapter nach einem der vorhergehenden Ansprüche, wobei der Spannhebel (30) zwei beiderseits an dem Spannkörper (10) und dem Spanndeckel (24) anliegende Wangen (32) aufweist.

5. Spannadapter nach Anspruch 4,
wobei der Spannhebel (30) mit an seinen Wangen (32) angeformten Lagerzapfen (38) in Lagerbohrungen (40) aufschnappbar ist.

6. Spannadapter nach einem der vorhergehenden Ansprüche, wobei die Spannbuchse (20) in einem Aufnahmeraum (16) des Spannkörpers (10) aufgenommen ist, wobei die Außenumfangskontur der Spannbuchse (20) mit der Innenumfangskontur des Aufnahmeraums (16) übereinstimmt.

7. Spannadapter nach Anspruch 6, wobei der Aufnahmeraum (16) und die Spannbuchse (20) sich vom proximalen Ende zunächst konisch erweitern und dann in einen zylindrischen distalen Abschnitt übergehen.

8. Spannadapter nach einem der vorhergehenden Ansprüche, wobei proximal anschließend an die Spannbuchse (20) eine Kontaktbuchse (52) in den Spannkörper (10) eingesetzt ist, die mit einem Steckverbinderanschluss für die elektrische Stimulation in Verbindung steht.

## Claims

1. Clamping adapter for a catheter, having a clamping body (10), having a bore (12) which passes axially through the clamping body (10) and which continues into a syringe attachment (14), having a shape-elastic clamping bush (20) which can be inserted into the clamping body (10), the inner channel (22) of which clamping bush communicates with the bore (12) of the clamping body (10) and the end of which clamping bush is supported axially in the clamping body (10), having a clamping lid (24) which can be placed onto the clamping body (10), has an insertion opening (28) aligned with the inner channel (22) of the clamping bush (20), is movable axially relative to the clamping body (10) and acts on the end face of the clamping bush (20), wherein the catheter (50) can be inserted through the insertion opening (28) of the clamping lid (24) into the inner channel (22) of the clamping bush and can be clamped in the inner channel (22) by axial compression of the clamping bush (20) by means of the clamping lid (24), wherein the clamping lid (24) is guided in a linearly displaceable manner relative to the clamping body (10), and having a clamping lever (30) which is pivotable between an open position and a clamping position about a pivot axis which runs transversely to the longitudinal axis of the clamping adapter, wherein the clamping lever (30) cooperates with the clamping lid in such a way that the clamping lid (24) releases the clamping bush (20) axially in the open position and compresses it axially in the clamping position, **characterized in that** the clamping body (10) continues proximally into the syringe attachment (14), **in that** the clamping bush (20) can be inserted distally into the clamping body (10), **in that** the inner channel (22) of the clamping bush (20) communicates with the distal end of the bore (12) of the clamping body (10), **in that** the proximal end of the clamping bush (20) is supported axially in the clamping body (10), **in that** the clamping lid (24) engages in a pot-shaped manner over the distal end of the clamping body (10) and engages with a coaxial pressing face (26) in the distal end of the clamping body (10) and bears against the distal end face of the clamping bush (20), **in that** the clamping lever (30) is mounted pivotably on the clamping lid (24) and moves the clamping lid (24) axially relative to the clamping body (10) by means of a sliding block guide (42, 44) that acts between the clamping lever (30) and the clamping body (10), **in that** the clamping lever (30) in its open position is moved away from the clamping adapter, wherein the clamping lid (24) is displaced axially in the distal direction relative to the clamping body (10), and **in that** the clamping lever (30) in its clamping position bears against the clamping adapter, wherein the clamping lid (24) is drawn axially in the proximal direction onto the clamping body (10).

2. Clamping adapter according to claim 1, wherein the sliding block guide has at least one guideway (42) which runs obliquely to the longitudinal axis of the clamping adapter and in which a guide pin (44) engages.

3. Clamping adapter according to claim 2, wherein the at least one guideway (42) is formed on the clamping lever (30) and the at least one guide pin (44) is formed on the clamping body (10).

4. Clamping adapter according to any of the preceding claims, wherein the clamping lever (30) has two cheeks (32) bearing against the clamping body (10) and the clamping lid (24) on both sides.

5. Clamping adapter according to claim 4, wherein the clamping lever (30) can be snapped into bearing bores (40) by bearing pins (38) integrally formed on its cheeks (32).

6. Clamping adapter according to any of the preceding claims, wherein the clamping bush (20) is accommodated in an accommodating space (16) of the clamping body (10), wherein the outer circumferential contour of the clamping bush (20) matches the inner circumferential contour of the accommodating space (16).

7. Clamping adapter according to claim 6, wherein the accommodating space (16) and the clamping bush (20) first expand conically from the proximal end and then merge into a cylindrical distal portion.

8. Clamping adapter according to any of the preceding claims, wherein, proximally adjoining the clamping bush (20), there is inserted into the clamping body (10) a contact bush (52) which is connected to a plug connector for electrical stimulation.

## Revendications

1. Adaptateur de serrage destiné à un cathéter comprenant un corps de serrage (10), un perçage (12) traversant axialement ce corps de serrage (10) et se prolongeant par un embout d'injection (14), une douille de serrage (20) à élasticité de forme pouvant être introduite dans le corps de serrage (10), dont le canal interne (22) communique avec le perçage (12) du corps de serrage (10) et dont l'extrémité s'appuie axialement dans ce corps de serrage (10), un capuchon de serrage (24) pouvant être positionné sur le corps de serrage (10), qui comporte une ouverture d'introduction (28) coïncidant avec le canal interne (22) de la douille de serrage, est mobile axialement par rapport au corps de serrage (10) et vient en prise sur la face frontale de la douille de serrage (20), le cathéter (50) pouvant être introduit, par l'ouverture d'introduction (28) du capuchon de serrage (24) dans le canal interne (22) de la douille de serrage, et pouvant être serré dans le canal interne (22) par compression axiale de la douille de serrage (20) au moyen du capuchon de serrage (24), le capuchon de serrage (24) étant susceptible de coulisser linéairement par rapport au corps de serrage (10), ainsi qu'un levier de serrage (30) susceptible de pivoter autour d'un axe de pivotement qui s'étend transversalement à l'axe longitudinal de l'adaptateur de serrage entre une position d'ouverture et une position de serrage, ce levier de serrage (30) coopérant avec le capuchon de serrage de sorte que le capuchon de serrage (24) détende axialement la douille de serrage (20) dans la position d'ouverture et la comprime axialement dans la position de serrage,
**caractérisé en ce que**
le corps de serrage (10) se prolonge à son extrémité proximale dans l'embout d'injection (14), la douille de serrage (20) peut être mise en place dans le corps de serrage (10) à son extrémité distale, le canal interne (22) de la douille de serrage (20) communique avec l'extrémité distale du perçage (12) du corps de serrage (10), l'extrémité proximale de la douille de serrage (20) s'appuie axialement dans le corps de serrage (10), le capuchon de serrage (24) vient en prise en forme de pot sur l'extrémité distale du corps de serrage (10) et vient en prise par une surface de compression coaxiale (26) dans l'extrémité distale du corps de serrage (10) et s'applique contre la face frontale distale de la douille de serrage (20), le levier de serrage (30) est articulé sur le capuchon de serrage (24) et déplace axialement le capuchon de serrage (24) par rapport au corps de serrage (10) au moyen d'un guidage à coulisse (42, 44) agissant entre le levier de serrage (30) et le corps de serrage (10), dans sa position d'ouverture le levier de serrage (30) est écarté de l'adaptateur de serrage, le capuchon de serrage (24) étant déplacé axialement en direction distale par rapport au corps de serrage (10) et, dans sa position de serrage, le levier de serrage (30) s'applique sur l'adaptateur de serrage, le capuchon de serrage (24) étant tiré axialement en direction proximale sur le corps de serrage (10).

2. Adaptateur de serrage conforme à la revendication 1,
dans lequel le guidage à coulisse comprend au moins un chemin de guidage (42) s'étendant obliquement par rapport à l'axe longitudinal de l'adaptateur de serrage dans lequel vient en prise une broche de guidage (44).

3. Adaptateur de serrage conforme à la revendication 2,
dans lequel le chemin de guidage (42) est situé sur le levier de serrage (30) et la broche de guidage (44) est située sur le corps de serrage (10).

4. Adaptateur de serrage conforme à l'une des revendications précédentes,
dans lequel le levier de serrage (30) comporte deux joues (32) s'appliquant de part et d'autre sur le corps de serrage (10) et sur le capuchon de serrage (24).

5. Adaptateur de serrage conforme à la revendication 4,
dans lequel le levier de serrage (30) peut s'enclencher dans des perçages de positionnement (40) par des broches de montage (38) formées sur ses joues (32).

6. Adaptateur de serrage conforme à l'une des revendications précédentes,
dans lequel le capuchon de serrage (20) est logé dans un volume de réception (16) du corps de serrage (10), le contour périphérique externe de la douille de serrage (20) coïncidant avec le contour périphérique interne du volume de réception (16).

7. Adaptateur de serrage conforme à la revendication 6,
dans lequel le volume de réception (16) et la douille de serrage (20) s'élargissent tout d'abord côniquement à partir de leur extrémité proximale puis se prolongent par un segment distal cylindrique.

8. Adaptateur de serrage conforme à l'une des revendications précédentes,
dans lequel à la suite de la douille de serrage (20) à son extrémité proximale est introduite dans le corps de serrage (10) une douille de contact (52) reliée à une prise de connexion par enfichage pour permettre la stimulation électrique.
